# FASCICULE DE BREVET EUROPEEN

(11) **EP 1 363 564 B1**
(45) Date de publication et mention de la délivrance du brevet: **20.04.2005**
(21) Numéro de dépôt: 02711744.9
(22) Date de dépôt: 27.02.2002
(51) Int. Cl.: A61F 2/42

(54) **PROTHESE DE POIGNET**
HANDGELENKPROTHESE
WRIST PROSTHESIS

(30) Priorité: 02.03.2001 CH 391012001
(43) Date de publication de la demande: 26.11.2003
(73) Titulaire: Guérard, Jean-Louis, 1025 St-Sulpice (CH); Pflug, Léopold, 1175 Lavigny (CH); Egloff, Daniel, 1003 Lausanne (CH)
(72) Inventeur: Guérard, Jean-Louis, 1025 St-Sulpice (CH); Pflug, Léopold, 1175 Lavigny (CH); Egloff, Daniel, 1003 Lausanne (CH)
(74) Mandataire: Ganguillet, Cyril
(86) Numéro de dépôt international: PCT/CH2002/000115
(87) Numéro de publication internationale: WO 2002/069853

(56) Documents cités:
- EP-A- 0 214 773
- EP-A- 0 454 645
- WO-A-95/22945
- WO-A-98/57600
- FR-A- 2 669 214
- FR-A- 2 681 240
- US-A- 4 100 626
- US-A- 4 229 840
- US-A- 4 307 473
- US-A- 4 714 476

## Description

La présente invention a pour objet une prothèse de poignet comprenant une pluralité de tiges rigides dont une tige centrale destinée à être ancrée dans le radius et deux tiges latérales destinées à être ancrées dans des métacarpiens, et un mécanisme d'articulation reliant lesdites tiges. Des prothèses de ce genre ont déjà été décrites notamment dans les publications de demandes de brevets français No 2681240 et 2669214, dans lesquelles les fonctions que doit remplir le mécanisme d'articulation sont expliquées. Dans le domaine technique des prothèses de poignet on cherche évidemment à fournir à la science médicale des dispositifs qui permettent aux patients d'effectuer, avec le poignet et les doigts, des mouvements aussi aisés et variés que possible, avec un confort satisfaisant.

Les prothèses de poignet connues à ce jour présentent des structures soit trop lâches soit trop rigides et sont souvent sujettes à descellement. Il faut par conséquent les remplacer généralement à très court terme.

Une prothèse de poignet selon le préambule de la revendication 1 est décrite dans US-A-4,307,473.

Le but de la présente invention est de proposer une prothèse de poignet qui permette d'une part de remédier aux problèmes des prothèses connues et d'autre part de mieux permettre les mouvements naturels du poignet, et avec un plus grand confort.

A cet effet, la présente invention concerne une prothèse de poignet comprenant une pluralité de tiges rigides, dont une tige centrale destinée à être ancrée dans le radius, et deux tiges latérales destinées à être ancrées dans des métacarpiens, et un mécanisme d'articulation reliant lesdites tiges, caractérisée en ce que le mécanisme d'articulation comporte trois éléments, solidaires respectivement des tiges latérales et de la tige centrale, assemblés élastiquement de manière à permettre des déplacements des tiges latérales par rapport à la tige centrale et des déplacements relatifs des tiges latérales l'une par rapport à l'autre.

Selon un mode d'exécution de ladite prothèse de poignet, ledit mécanisme d'articulation comporte un siège en un matériau rigide avec deux faces frontales, un axe en un matériau élastiquement déformable et extensible, engagé dans une ouverture centrale du siège, chacune des tiges latérales comprenant une embase comportant un passage transversal, lesdites embases présentant des surfaces frontales de formes conjuguées à celles des faces frontales du siège, l'axe étant maintenu en extension par verrouillage de ses extrémités dans les passages transversaux des embases par des verrous de sorte que lesdites faces frontales des embases sont pressées contre les faces frontales du siège de façon à constituer des articulations de type rotulien permettant des déplacements relatifs limités des tiges latérales par rapport au siège.

Lesdites faces frontales du siège peuvent être de forme concave, les dites faces frontales des embases ayant la forme de calottes sphériques. Selon une variante, lesdites faces frontales du siège sont de forme convexe, lesdites faces frontales des embases étant de forme concave.

La tige centrale peut être engagée par une extrémité dans une forure transversale ménagée au milieu de la longueur du siège et présenter à ladite extrémité une fente qui enfourche la partie centrale de l'axe; la partie centrale de l'axe et la fente de la tige centrale pouvant présenter des profils correspondants agencés pour assurer la fixation de ladite tige par rapport à l'axe.

Le siège peut être de forme cylindrique, la tige centrale étant rectiligne et disposée radialement par rapport à l'axe du siège et les tiges latérales comportant un jambage rectiligne solidaire de l'embase, et décalé latéralement par rapport à l'axe de l'embase.

Les embases des tiges latérales peuvent être de forme annulaire avec un passage central muni d'un épaulement interne destiné à retenir un dispositif de verrouillage qui fixe dans l'embase une des extrémités de l'axe en maintenant ce dernier en extension.

Chaque dispositif de verrouillage peut être formé de deux demi-verrous maintenus serrés l'un contre l'autre par leur engagement dans ledit passage central et retenus en appui contre ledit épaulement par la traction de l'axe; les dispositifs de verrouillage pouvant être bloqués contre tout mouvement de rotation par rapport à l'embase qui les contient par une goupille traversant l'embase et l'un des demi-verrous.

On décrit ci-après, à titre d'exemple, une forme d'exécution de la prothèse de poignet selon l'invention en se référant au dessin annexé dont :
La fig. 1 est une vue en coupe d'une tige latérale de la prothèse,
La fig. 2 une vue en élévation dans le sens de la flèche A, de la tige de la fig. 1,
La fig. 3 une vue en perspective éclatée des différents constituants de la prothèse,
La fig. 4 une vue en élévation de l'ensemble de la prothèse à l'état assemblé,
La fig. 5 une vue en plan de dessus de la prothèse de la fig. 4,
La fig. 6 une vue en coupe selon la ligne 6 - 6 de la fig. 5 montrant tous les constituants de la prothèse, et
La fig. 7 est une vue en perspective de la prothèse à l'état assemblé.

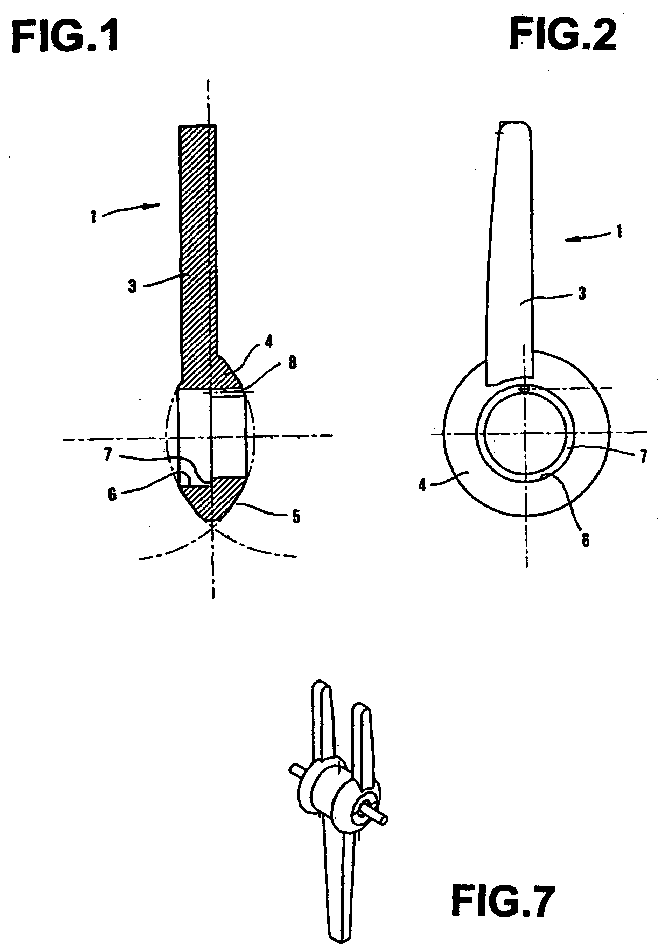

La prothèse se compose essentiellement de trois tiges rigides, dont deux tiges latérales 1 et 2 et une tige centrale 9, assemblées de manière à être mobiles les unes par rapport aux autres. Les fig. 1 et 2 représentent la tige latérale 1 qui est symétrique de la tige 2 et formée d'une seule pièce métallique ou en matière plastique, mise en forme par moulage ou par usinage. Cette pièce comporte un jambage 3 rectiligne, de section rectangulaire (ou de tout autre forme adéquate, telle que polygonale, circulaire ou ovale), présentant un profil légèrement effilé à partir d'une embase 4. Cette partie 4 de la tige 1 est de forme annulaire vue en élévation (fig. 2) et de forme ovoide vue en coupe (fig. 1). En particulier la face frontale 5 située à l'opposé de celle qui est visible à la fig. 2 est une portion de surface sphérique traitée de manière à être parfaitement régulière et lisse. L'embase de chacune des tiges latérales joue le rôle d'une rotule. L'ouverture centrale de l'embase annulaire 4 est un passage cylindrique 6 qui présente un épaulement interne 7, la partie arrière du passage 6 ayant un diamètre légèrement plus grand que la partie avant. Une forure 8 dont le diamètre est égal à la largeur de l'épaulement 7 est ménagée le long de la génératrice supérieure du passage 6, dans la zone avant ayant le diamètre le plus petit.

Considérant la fig. 2 on voit que le jambage 3 est placé dans une position légèrement décalée par rapport à un axe vertical passant par le centre de l'embase 4. Pour la tige latérale 2 qui a la même forme et la même constitution générale que la tige 1, ce décalage du jambage 3 est symétrique par rapport à ce que montre la fig. 2, comme on le verra encore plus loin.

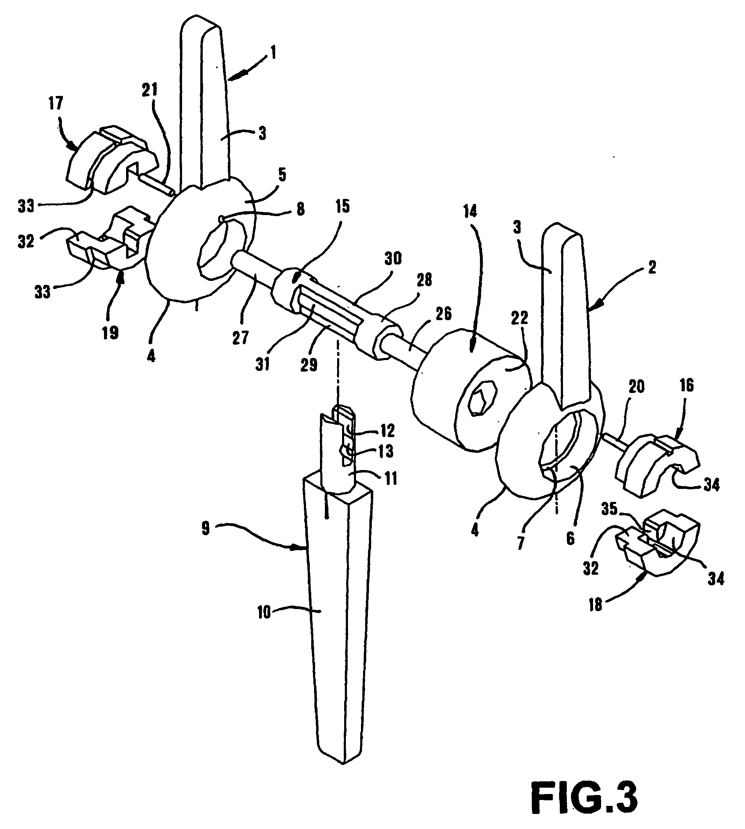

Les deux tiges 1 et 2 sont représentées à la fig. 3 dans des positions analogues à celles qu'elles occupent dans la prothèse montée et on voit qu'elles se font face, la surface 5 de la pièce 1 étant visible à la fig. 3 alors que cette même surface 5 est cachée en ce qui concerne la pièce 2.

La troisième tige 9 est également visible à la fig. 3. Elle comporte un jambage 10 de section rectangulaire allant en décroissant vers le bas et qui se prolonge vers le haut par un tourillon 11 cylindrique dans lequel est fraisée un fente 12, à flancs parallèles, interrompus à mi-hauteur par une gorge transversale 13.

Les moyens mécaniques prévus pour assembler et ajuster les unes par rapport aux autres les trois tiges 1, 2 et 9 consistent en un siège 14, un axe 15, deux paires de demi-verrous 16, 17, 18 et 19, et deux goupilles 20 et 21, attribuées chacune à un des demi-verrous de chaque paire.

Il convient de préciser que le dessin de la figure 3 a été obtenu à l'aide d'un programme de dessin mal adapté pour la représentation des courbes qui sont dessinées sous forme de lignes polygonales. En réalité, la forme extérieure des pièces 17, 19, 4, 27, 15, 28, 26, 14, 16 et 18 est circulaire, de même que les passages qui les traversent.

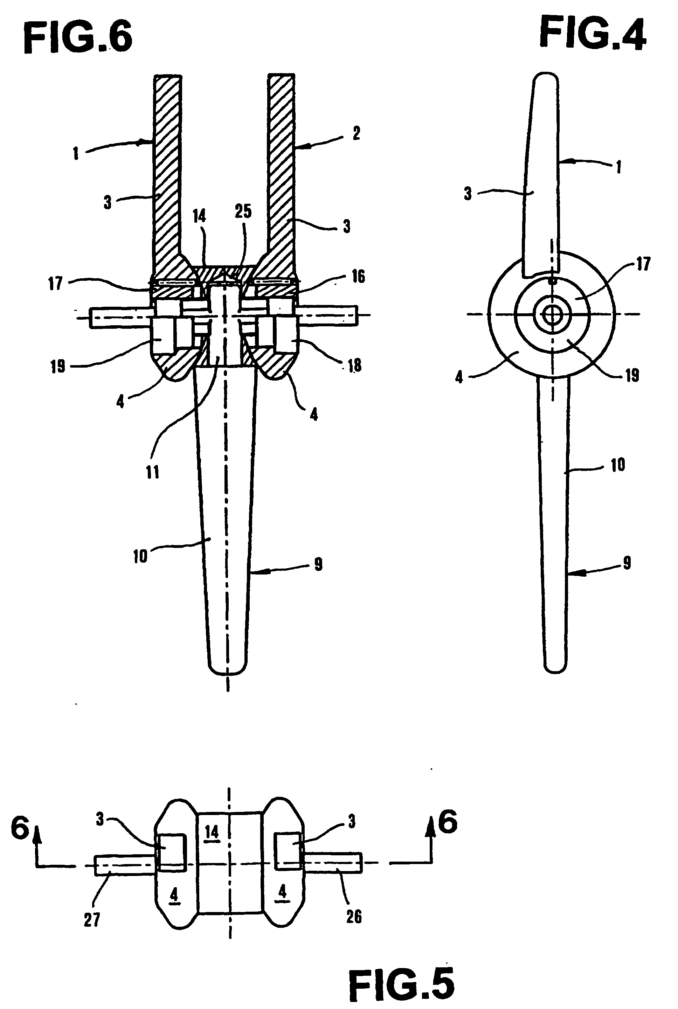

Le siège 14 est une pièce de forme extérieure cylindrique, avec deux faces extrêmes 22, 23 en portions de surfaces sphériques concaves, exactement ajustées de manière à recevoir chacune la face 5 de l'une des tiges 1 ou 2. Il est percé selon son axe d'un passage cylindrique 24 et présente enfin une forure borgne 25 (fig. 6) qui le traverse de bas en haut verticalement jusqu'au voisinage de sa surface périphérique extérieure. Le diamètre de cette forure est suffisant pour donner passage au tourillon 11 de la tige centrale 9, la gorge 13 venant se placer au centre du passage cylindrique 24.

Le siège peut être réalisé en matière synthétique, par exemple en polyéthylène implantable présentant une bonne surface de glissement.

Selon une variante, le siège peut être réalisé avec des faces extrêmes 22, 23 convexes. Dans ce cas, les embases 4 des tiges 1 et 2 seront réalisées avec des surfaces frontales 5 concaves agencées pour correspondre aux surfaces convexes des faces extrêmes du siège, de façon à permettre la réalisation d'articulations de type rotulien.

L'axe 15 est la pièce essentielle de tout le mécanisme d'assemblage. Cette pièce est en élastomère ou autre matière synthétique souple présentant les degrés de stabilité et d'élasticité voulus. Sa forme générale est cylindrique avec deux tigerons 26, 27 à ses extrémités, entre ces tigerons une partie centrale cylindrique 28 dont le diamètre est tel qu'elle pénètre dans le passage cylindrique 24 du siège 14, et qui présente deux échancrures 29 et 30 avec des fonds plats et parallèles et un bourrelet 31 allongé au centre de chaque fond d'une échancrure. La distance entre les fonds parallèles des échancrures est égale à la largeur de la fente 12 et les rayons des bourrelets 31 correspondent à celui de la gorge 13. On voit à la fig. 3 que le tourillon 11 de la tige 9 en s'engageant sur la partie centrale de l'axe 15, vient se clipser sur les bourrelets 31 et sera maintenu en place par l'élasticité de la matière de l'axe 15.

L'élasticité de cette matière assure également le serrage des faces sphériques 5 des tiges 1 et 2 contre les faces concaves 22 et 23 du siège 14. Pour cela les tigerons 26 et 27 servent de moyens de traction pour réaliser une extension de la partie cylindrique 28 de l'axe 15 de manière à permettre la mise en place des paires de demi-verrous 16, 18 et 17, 19.

Les demi-verrous 16, 18 et 17, 19 sont des pièces en arcs de cylindre limités par une face plane 32 de manière que deux demi-verrous homologues (par exemple 16, 18) placés l'un contre l'autre avec leurs faces planes 32 en contact forment un corps cylindrique à deux étages de diamètres différents, séparés par un épaulement 33. Intérieurement chaque assemblage de deux demi-verrous homologues présente une creusure cylindrique 34 avec dans son fond une ouverture profilée 35 dont les dimensions correspondent au profil de la partie centrale échancrée de l'axe 15. Ainsi, notamment, dans chaque demi-verrou les angles entre la face plane 32 et les bords de l'échancrure rectangulaire 35 sont biseautés de manière à correspondre avec le bourrelet 31 de l'axe 15. Enfin les deux demi-verrous 16 et 17 présentent dans leur face latérale externe, à la partie supérieure de celle-ci, une gorge 36 parallèle à l'axe et dont le diamètre correspond à celui des goupilles 20 et 21.

Les fig. 4, 5, 6 et 7 montrent comment la prothèse se présente dans son état assemblé. L'élasticité de l'axe 15 maintient les deux tiges latérales 1 et 2 et la tige centrale 9 liées au siège 14 en leur permettant divers mouvements. L'axe 15 étant en extension, les flancs d'extrémité des échancrures 29 et 30 sont accrochés aux bords des échancrures 35 dans les fonds des creusures 34 des verrous 16/18 et 17/19 qui sont eux-mêmes engagés dans les passages 6 des embases 4 des tiges 1 et 2. Les faces sphériques 5 des embases 4 sont donc maintenues appuyées contre les faces concaves 22 et 23 du siège 14, alors que le tourillon 11 de la tige centrale 9 est engagé dans le trou borgne 25 du siège, la partie centrale de la zone cylindrique 28 de l'axe 15 s'étend dans la fente 12 et les bourrelets 31 sont engagés dans les gorges 13. Les goupilles 20 et 21 solidarisent en rotation les verrous 16/18 et 17/19 chacun avec la tige latérale 1 ou 2 à laquelle il est associé.

Cette disposition permet des mouvements de rotation des tiges 1 et 2 par rapport au siège 14 autour de l'axe de ce dernier ainsi que certains mouvements de pivotement des tiges 1 et 2 autour d'axes perpendiculaires à celui du siège. Elle permet également des mouvements de rotation de la tige centrale 9 autour de son axe propre, du fait que les échancrures 29 et 30 ont une longueur supérieure au diamètre du tourillon 11 (voir fig. 6).

Aux fig. 3, 5 et 6 on a représenté l'axe 15 encore pourvu de ses tigerons 26 et 27, mais il importe de noter que ces éléments ne remplissent qu'une fonction auxiliaire au moment du montage de la prothèse et seront coupés après la mise en place.

En effet, pour réaliser l'opération de montage avec les éléments décrits, une des paires de demi-verrous, la tige latérale, le siège et l'axe seront assemblés, les deux demi-verrous étant appuyés l'un contre l'autre de manière à enfermer entre eux l'extrémité correspondante cylindrique de la partie centrale 28 de l'axe 15. Ensuite, l'autre tige latérale sera mise en place, la surface sphérique 5 de son embase étant placée en contact avec la face concave correspondante du siège. L'axe 15 étant alors mis en extension par traction sur les tigerons 26, 27, de manière que l'autre extrémité cylindrique de la partie centrale 28 de l'axe apparaisse sur l'extérieur du passage 6 de la seconde tige latérale, la seconde paire de demi-verrous pourra être engagée de part et d'autre de la partie saillante de l'axe et serrée de manière à enfermer dans la creusure 34 l'autre extrémité de la partie centrale 28 de l'axe 15. Il suffit finalement de relâcher la tension sur les tigerons 26/27 pour que l'assemblage soit en place. La tige centrale 9 peut être engagée dans la forure transversale 25 du siège 14 le tourillon 11 étant fixé par sa gorge 13 sur le bourrelet 31 de l'axe.

Ces opérations étant exécutées, les tigerons 26/27 ne sont plus d'aucune utilité et peuvent être sectionnés.

La prothèse selon la présente invention permet de mieux reproduire la mobilité des os les uns par rapport aux autres que les prothèses de l'art antérieur, notamment grâce à sa souplesse due aux divers degrés de liberté qu'elle autorise en combinaison avec les propriétés élastiques de son axe 15, qui à chaque degré de liberté associe une force de rappel correspondante.

Les principaux degrés de liberté qu'autorise la prothèse selon l'invention sont les suivants :
- rotation des tiges 1 et 2 autour de l'axe 15
- pivotement latéral des tiges 1 et 2, avec décollement possible de leurs embases 4 par rapport au siège 14 en cas de mouvement extrême
- mouvement axial longitudinal de la tige 9.

## Revendications

1. Prothèse de poignet comprenant une pluralité de tiges rigides (1, 2, 9), dont une tige,centrale (9) destinée à être ancrée dans le radius, et deux tiges latérales (1, 2) destinées à être ancrées dans des métacarpiens, et un mécanisme d'articulation (4, 5, 14, 15, 11, 16, 17, 18, 19) reliant lesdites tiges, **caractérisée en ce que** le mécanisme d'articulation comporte trois éléments (4, 4, 11), solidaires respectivement des tiges latérales (1, 2) et de la tige centrale (9), assemblés élastiquement de manière à permettre des déplacements des tiges latérales par rapport à la tige centrale et des déplacements relatifs des tiges latérales l'une par rapport à l'autre.

2. Prothèse selon la revendication 1, **caractérisée en ce que** ledit mécanisme d'articulation comporte un siège (14) en un matériau rigide avec deux faces frontales (22, 23), un axe (15) en un matériau élastiquement déformable et extensible, engagé dans une ouverture centrale du siège, **en ce que** chacune des tiges latérales (1, 2) comprend une embase (4) comportant un passage transversal (6), lesdites embases (4) présentant des surfaces frontales (5) de formes conjuguées à celles des faces frontales (22, 23) du siège, et **en ce que** l'axe est maintenu en extension par verrouillage de ses extrémités dans les passages transversaux (6) des embases par des verrous (16/18, 17/19) de sorte que lesdites faces frontales (5) des embases (4) sont pressées contre les faces frontales (22, 23) du siège de façon à constituer des articulations de type rotulien permettant des déplacements relatifs limités des tiges latérales par rapport au siège.

3. Prothèse selon la revendication 2, **caractérisée en ce que** lesdites faces frontales (22, 23) du siège sont de forme concave, lesdites faces frontales (5) des embases ayant la forme de calottes sphériques.

4. Prothèse selon la revendication 2, **caractérisée en ce que** lesdites faces frontales (22, 23) du siège sont de forme convexe, lesdites faces frontales (5) des embases étant de forme concave.

5. Prothèse selon l'une des revendications précédentes, **caractérisée en ce que** la tige centrale (9) est engagée par une extrémité (11) dans une forure transversale (25) ménagée au milieu de la longueur du siège (14) et présente à ladite extrémité (11) une fente (12) qui enfourche la partie centrale (28) de l'axe (15).

6. Prothèse selon la revendication 5, **caractérisée en ce que** la partie centrale (28) de l'axe (15) et la fente (12) de la tige centrale (9) présentent des profils correspondants agencés pour assurer la fixation de ladite tige par rapport à l'axe.

7. Prothèse selon l'une quelconque des revendications précédentes, **caractérisée en ce que** le siège (14) est de forme cylindrique, la tige centrale (9) est rectiligne et disposée radialement par rapport à l'axe du siège et les tiges latérales (1, 2) comportent un jambage (3) rectiligne solidaire de l'embase (4) et décalé latéralement par rapport à l'axe de l'embase.

8. Prothèse selon l'une quelconque des revendications précédentes, **caractérisée en ce que** les embases (4) des tiges latérales (1, 2) sont de forme annulaire avec un passage central (6) muni d'un épaulement interne (7) destiné à retenir un dispositif de verrouillage (16 - 19) qui fixe dans l'embase une des extrémités de l'axe (15) en maintenant ce dernier en extension.

9. Prothèse selon la revendication 8, **caractérisée en ce que** chaque dispositif de verrouillage est formé de deux demi-verrous (16/18, 17/19) maintenus serrés l'un contre l'autre par leur engagement dans ledit passage central (6) et retenus en appui contre ledit épaulement (7) par la traction de l'axe (15) .

10. Prothèse selon la revendication 9, **caractérisée en ce que** les dispositifs de verrouillage (16/18, 17/19) sont bloqués contre tout mouvement de rotation par rapport à l'embase qui les contient par une goupille (20, 21) traversant l'embase et l'un des demi-verrous.

## Patentansprüche

1. Handgelenkprothese mit mehreren starren Stangen (1, 2, 9), von denen eine zentrale Stange (9) dazu bestimmt ist, an dem Radius verankert zu werden, und zwei seitliche Stangen dazu bestimmt sind, an dem Metacarpus verankert zu werden, und mit einem Gelenkmechanismus (4, 5, 14, 15, 11, 16, 17, 18, 19), der die Stangen miteinander verbindet, **dadurch gekennzeichnet, dass** der Gelenkmechanismus drei Elemente (4, 4, 11) aufweist, die mit den seitlichen Stangen (1, 2) bzw. der zentralen Stange (9) fest verbunden und elastisch zusammengefügt sind, derart, dass Versetzungen der seitlichen Stangen in Bezug zu der zentralen Stange und relative Versetzungen der seitlichen Stangen in Bezug zueinander erlaubt sind.

2. Prothese nach Anspruch 1, **dadurch gekennzeichnet, dass** der Gelenkmechanismus einen Sitz (14) aus einem starren Material mit zwei Stirnseiten (22, 23) und eine Achse (5) aus einem elastisch deformierbaren und dehnbaren Material aufweist, die in eine zentrale Öffnung des Sitzes eingreift, dass jede der seitlichen Stangen (1, 2) eine Basis (4) mit einer transversalen Passage (6) aufweist, wobei diese Basen Stirnflächen (5) mit einer den Stirnseiten (22, 23) des Sitzes zugeordneten Form aufweisen, und dass die Achse in Dehnung durch eine Verriegelung ihrer Enden in den transversalen Passagen (6) der Basen durch Riegel (16/18, 17/19) gehalten ist, derart, dass die Stirnflächen (5) der Basen (4) gegen die Stirnseiten (22, 23) des Sitzes gepresst werden, um Gelenkbewegungen nach Art eines Kugelgelenkes zu ermöglichen, um begrenzte relative Versetzungen der seitlichen Stangen in Bezug zu dem Sitz zu erlauben.

3. Prothese nach Anspruch 2, **dadurch gekennzeichnet, dass** die Stirnseiten (22, 23) des Sitzes eine konkave Form und die Stirnflächen (5) der Basen die Form sphärischer Kalotten aufweisen.

4. Prothese nach Anspruch 2, **dadurch gekennzeichnet, dass** die Stirnseiten (22, 23) des Sitzes eine konvexe Form und die Stirnflächen (5) der Basen eine konkave Form aufweisen.

5. Prothese nach einem der vorhergehenden Ansprüche, **dadurch gekennzeichnet, dass** die zentrale Stange (9) mit einem Ende (11) in eine transversale, in der Mitte der Länge des Sitzes (14) angeordnete Bohrung (25) eingreift und an diesem Ende (11) einen Schlitz (12) aufweist, der den zentralen Teil (28) der Achse (15) nach Art einer Gabel umgreift.

6. Prothese nach Anspruch 5, **dadurch gekennzeichnet, dass** der zentrale Teil (28) der Achse (15) und der Schlitz (12) der zentralen Stange (9) korrespondierende Profile aufweisen, die eingerichtet sind, um die Fixierung dieser Stange in Bezug zu der Achse sicher zu stellen.

7. Prothese nach einem der vorhergehenden Ansprüche, **dadurch gekennzeichnet, dass** der Sitz (14) eine zylindrische Form hat, die zentrale Stange (9) gerade ist und radial in Bezug zu der Achse des Sitzes angeordnet ist, und die seitlichen Stangen (1, 2) einen geraden Stab (3) aufweisen, der fest mit der Basis (4) verbunden und seitlich in Bezug zu der Achse der Basis versetzt ist.

8. Prothese nach einem der vorhergehenden Ansprüche, **dadurch gekennzeichnet, dass** die Basen (4) der seitlichen Stangen (1, 2) die Form eines Ringes mit einer zentralen Passage (6) aufweisen, die mit einer internen Schulter (7) versehen ist, die zum Halten einer Verriegelungseinrichtung (16 - 19) dient, welche in der Basis eines der Enden der Achse (15) fixiert, indem dieses in Dehnung gehalten wird.

9. Prothese nach Anspruch 8, **dadurch gekennzeichnet, dass** jede Verriegelungseinrichtung aus zwei Halbriegeln (16/18, 17/19) gebildet ist, die durch ihre gegenseitige Anlage in der zentralen Passage (6) eingeklemmt gehalten und in Anlage gegen die Schulter (7) durch den Zug der Achse (15) festgehalten sind.

10. Prothese nach Anspruch 9, **dadurch gekennzeichnet, dass** die Verriegelungseinrichtungen (16/18, 17/19) gegen jede Drehbewegung in Bezug zu der sie aufnehmenden Basis durch einen Stift (20, 21) blockiert sind, der die Basis und einen der Halbriegel durchdringt.

## Claims

1. Wrist prosthesis comprising a plurality of rigid rods (1, 2, 9), including a central rod (9) intended to be anchored in the radius and two lateral rods (1, 2) intended to be anchored in metacarpals, and an articulation mechanism (4, 5, 14, 15, 11, 16, 17, 18, 19) connecting the said rods, **characterised in that** the articulation mechanism comprises three elements (4, 4, 11), secured respectively to the lateral rods (1, 2) and central rod (9), assembled elastically so as to allow movements of the lateral rods with respect to the central rod and relative movements of the lateral rods with respect to each other.

2. Prosthesis according to claim 1, **characterised in that** the said articulation mechanism comprises a seat (14) made from a rigid material with two front faces (22, 23), a spindle (15) made from an elastically deformable and extensible material, engaged in a central opening in the seat, **in that** each of the lateral rods (1, 2) comprises a base (4) comprising a transverse passage (6), the said bases (4) having front surfaces (5) with shapes conjugate with those of the front faces (22, 23) of the seat, and **in that** the spindle is held in extension by locking of its ends in the transverse passages (6) in the bases by locks (16/18, 17/19) so that the said front faces (5) of the bases (4) are pressed against the front faces (22, 23) of the seat so as to constitute articulations of the swivel type allowing limited relative movements of the lateral rods with respect to the seat.

3. Prosthesis according to claim 2, **characterised in that** the said front faces (22, 23) of the seat are concave in shape, the said front faces (5) of the bases having the form of spherical caps.

4. Prosthesis according to claim 2, **characterised in that** the said front faces (22, 23) of the seat are convex in shape, the said front faces (5) of the bases being concave in shape.

5. Prosthesis according to one of the preceding claims, **characterised in that** the central rod (9) is engaged by one end (11) in a transverse bore (25) provided half-way along the seat (14) and has at the said end (11) a slot (12) which straddles the central part (28) of the spindle (15).

6. Prosthesis according to claim 5, **characterised in that** the central part (28) of the spindle (15) and the slot (12) in the central rod (9) have corresponding profiles arranged so as to provide the fixing of the said rod with respect to the spindle.

7. Prosthesis according to any one of the preceding claims, **characterised in that** the seat (14) is cylindrical in shape, the central rod (9) is rectilinear and disposed radially with respect to the axis of the seat and the lateral rods (1, 2) comprise a rectilinear jamb (3) fixed to the base (4) and offset laterally with respect to the axis of the base.

8. Prosthesis according to any one of the preceding claims, **characterised in that** the bases (4) of the lateral rods (1, 2) are annular in shape with a central passage (6) provided with an internal shoulder (7) intended to hold a locking device (16-19) which fixes in the base one of the ends of the spindle (15) whilst keeping the latter in extension.

9. Prosthesis according to claim 8, **characterised in that** each locking device is formed by two half-locks (16/18, 17/19) kept clamped against each other by their engagement in the said central passage (6) and held in abutment against the said shoulder (7) by the traction of the spindle (15).

10. Prosthesis according to claim 9, **characterised in that** the locking devices (16/18, 17/19) are locked against any rotation movement with respect to the base which contains them by a pin (20, 21) passing through the base and one of the half-locks.
